# EUROPEAN PATENT SPECIFICATION

(11) **EP 2 056 783 B1**
(45) Date of publication and mention of the grant of the patent: **22.11.2017**
(21) Application number: 07837604.3
(22) Date of filing: 30.08.2007
(51) Int. Cl.: A61Q 5/00, A61Q 5/12, A61Q 7/02, G06Q 30/02, A61K 8/42, A61K 8/49, A61Q 7/00, A61K 8/67

(54) **HAIR CARE COMPOSITIONS THAT CAN INCREASE THE APPEARANCE OF THICKER AND FULLER HAIR**
HAARPFLEGEZUSAMMENSETZUNG ZUR ERZEUGUNG DES EINDRUCKS VON DICKEREM UND VOLLEREM HAAR
COMPOSITIONS, PROCÉDÉS ET ARTICLES COMMERCIAUX DE SOINS CAPILLAIRES CAPABLES DE DONNER PLUS DE VOLUME ET D'ÉPAISSEUR AUX CHEVEUX

(30) Priority: 30.08.2006 US 841095 P
(43) Date of publication of application: 13.05.2009
(60) Divisional application: 12186255.1
(73) Proprietor: The Procter & Gamble Company, Cincinnati, OH 45202 (US)
(72) Inventor: DAWSON, Thomas, Larry, Jr., Hamilton, Ohio 45011 (US); OBLONG, John, Erich, Loveland, Ohio 45140 (US); YOUNGQUIST, Robert, Scott, Mason, Ohio 45040 (US); XIE, Sancai, West Chester, Ohio 45069 (US); EICKHOFF, David Joseph, Ft. Mitchell, Kentucky 41017 (US)
(74) Representative: Sauvaître, Thibault Bruno
(86) International application number: PCT/US2007/019180
(87) International publication number: WO 2008/027541

(56) References cited:
- EP-A- 1 683 509
- WO-A-99/66887
- WO-A-02/087700
- DE-A1-102005 010 142
- FR-A- 2 751 541
- JP-A- 2001 002 532
- DATABASE MEDLINE [Online] US NATIONAL LIBRARY OF MEDICINE (NLM), BETHESDA, MD, US; May 2001 (2001-05), DE LACHARRIÈRE O ET AL: "Hair diameter diversity: a clinical sign reflecting the follicle miniaturization." XP002468228 Database accession no. NLM11346342

## Description

### CROSS REFERENCE TO RELATED APPLICATION

This Application claims the benefit of U.S. Provisional Application Serial No. 60/841,095 filed August 30, 2006.

### FIELD OF THE INVENTION

The present invention relates to hair care compositions, methods, and articles of commerce that can increase the appearance of thicker and/or fuller hair. The present invention also relates to methods of marketing such compositions.

### BACKGROUND OF THE INVENTION

Many attributes contribute to the appearance of hair considered to be attractive. For instance, hair with a full and thick appearance is very desirable, whether it be on the scalp, beard, or moustache regions. In contrast, hair with a thin appearance is not as attractive, and can even lead to a perception that the thin-haired individual is older than their chronological age. Furthermore, thin hair can be more difficult to style, and typically cannot be styled into as many hairstyles, leaving the individual frustrated and with an unkempt appearance. Because of the foregoing problems associated with thin hair, many thin-haired individuals expend great effort and time on grooming, yet still do not attain their desired hairstyle and appearance. This can lead to frustration and/or lack of confidence in his or her appearance. These problems can be experienced by both female and male consumers.

Accordingly, there is a need to provide consumers with a way to increase the fullness and thickness of hair appearance, thus resulting in younger-looking, more attractive hair appearance.

### SUMMARY OF THE INVENTION

The present invention relates to hair care compositions, methods, and articles of commerce that can help increase the appearance of fuller and/or thicker of hair, thus resulting in younger-looking hair, by increasing the diameter of hair shafts and follicles. The invention also relates to methods of marketing.

In one aspect, a method comprises topically applying a hair care composition comprising an effective amount of a hair growth inhibiting agent to the scalp and/or face (e.g., beard region) of a mammal for the purpose of increasing the appearance of fuller and/or thicker hair. The hair growth inhibiting agent may comprise a material that up-regulates aquaporin-3 expression.

The hair care composition comprises a synergistic mixture of a xanthine compound, preferably caffeine, a vitamin B3 compound, preferably niacinamide, and a panthenol compound, preferably panthenol.. The composition comprises from 0.1% to 10% of caffeine, preferably from 0.5% to 5% of caffeine, more preferably from 1% to 2% of caffeine. The composition comprises from 0.1% to 25% of niacinamide, preferably from 0.5% to 15% of niacinamide, more preferably from 3.5% to 7.5% of niacinamide. The composition comprises from 0.01% to 3% of panthenol, preferably from 0.02% to 1% of panthenol, more preferably from 0.2% to 0.5% of panthenol. The composition comprises from 10-90% alcohol, preferably 15-75% alcohol, more preferably from 25-50% alcohol, wherein said alcohol is ethanol. The composition can optionally comprise any other suitable ingredients as desired. The composition may also comprise a thickener that helps to hold the active agents on the scalp, providing substantivity to the composition, such that it does not drip undesirably onto unintended areas of the body, clothing, or home furnishings.

Use of the composition of the present invention for increasing the hair shaft diameter to give the appearance of thicker and fuller hair.

These and other features, aspects, and advantages of the present invention will become evident to those skilled in the art from a reading of the present disclosure.

### DETAILED DESCRIPTION OF THE INVENTION

While the specification concludes with the claims particularly pointing and distinctly claiming the invention, it is believed that the present invention will be better understood from the following description.

In the past, various compositions comprising hair growth inhibition agents have been applied to body areas where hair is not desired, in an effort to eliminate, decrease, and/or slow the growth of unwanted hair. Contrary to this conventional wisdom, applicants have found that topical application of hair growth inhibitors to regions where the appearance of more hair is desired (e.g., scalp and/or beard area) can actually increase the appearance of thicker and/or fuller hair.

Although not wishing to be limited by theory, it is believed by applicants that topical application of various hair growth inhibitors stimulates aquaporin 3 ("AQP3") up-regulators, which in turn results in thicker hair shafts and follicles. This increase in the diameter of the hair shafts and follicles leads to the appearance of hair that is thicker and fuller, thus resulting in the appearance of fuller and/or thicker hair. Furthermore, it leads to the appearance of younger looking hair, since hair diameter is known to decrease with one's chronological age. (Olsen, EA et al. Evaluation and treatment of male and female pattern hair loss. J Am Acad Dermatol Vol. 52, Number 2, Feb 2005 pg. 305.); (Birch-MP et al. Hair density, hair diameter and the prevalence of female pattern loss. British Journal of Dermatology 2001; 144: 297-304.); (Courtois-M et al. Ageing and hair cycles. British Journal of Dermatology 1995; 132:86-93. - L'Oreal); (Lacarrubba-F et. al. Videodermatoscopy Enhances Diagnostic Capability in Some Forms of Hair Loss. Am J Clin Dermatol 2004; 5 (3): 205-208); (De Lacharriere-O et al. Hair Diameter Diversity. Arch Dematol. 2001; 137:641-646 L'Oreal); (Hoffman-R. Trichoscan: combining epiluminescence microscopy with digital image analysis for the measurement of hair growth in vivo. Eur J Drmatol 2001; 11:362-8.)

Aquaporins ("AQP") are a family of water-channel proteins found in the plasma membrane. Currently, 13 AQP have been identified in humans, and they are classified into two groups: aquaporins and aquaglyceroporins. Most of the identified aquaporins belong to the aquaporin group that is selective primarily for the passage of water. AQP 3, 7, 9, and 10 belong to the aquaglyceroporin group that facilitates the movement of water, glycerol, and various other solutes.

Through immunocytochemistry, applicants have found that AQP3 is strongly expressed in the proliferating keratinocytes of the hair follicles. In addition, applicants have also found that AQP3 immunostaining can be detected in the hair shaft. It is believed by applicants that AQP3 up-regulators increase the thickness of hair fibers and follicles by stimulating AQP3 expression, which allows more water and water-binding molecules to be transported into the cells, thus improving cellular metabolism and increasing cellular size. This leads to increased hydration of the hair's cuticle layer, which expands the cuticle, making it thicker. As a result, the diameter of the hair fiber and of the hair follicle increase, resulting in the appearance of thicker, fuller hair.

Because consumers are not familiar with the use of hair growth inhibiting agents for the purpose of increasing the appearance of thicker and fuller hair, the present invention also provides methods of marketing that can be advantageously used to help potential consumers appreciate the benefits that they can derive from such a product and/or its method of use. Furthermore, a method of marketing a first composition by comparing it to a second composition that comprises a hair growth inhibition agent is also provided. This method helps consumers to identify products that can potentially give similar benefits.

All percentages, parts and ratios are based upon the total weight of the hair care compositions of the present invention and all measurements made are at 25°C, unless otherwise specified. All such weights as they pertain to listed ingredients are based on the active level and, therefore, do not include carriers or by-products that may be included in commercially available materials, unless otherwise specified.

As used herein, the term "hair care compositions" are compositions that are applied to the hair and/or the skin underneath the hair, including compositions used to treat or care for the hair. Products contemplated by the phrase "hair care composition" include, but are not limited to liquids, creams, wipes, hair conditioners (rinse-off and leave-on), hair tonics, shampoos, hair colorants, mousses, propellant lotions, emulsions, shave gels, after-shave tonics and lotions, temporary beard hair dyes, and the like.

"Hair growth inhibiting agent" or "hair growth inhibition agent" includes any material that can reduce, inhibit, attenuate, or diminish mammalian hair growth.

"Increase the appearance of fuller and thicker hair" means the diameters of hair follicles and/or shafts in the subject region of hair (e.g., scalp, beard) are increased by a statistically significant amount, when an effective amount of a composition of the present invention is topically applied to the desired region over a result-effective period of time.

"Mammalian hair," as referenced herein, includes hair on any part of the body of a mammal, and can include but is not limited to facial, cranial, or body hair. For instance, it can include hair on the scalp, head, neck, beard, moustache, eyebrows and sideburns hair.

The term "topical application," as used herein, means to apply or spread the compositions of the present invention onto the surface of the keratinous tissue from which the hair to be affected grows.

The term "dermatologically-acceptable," as used herein, means that the compositions or components thereof so described are suitable for use in contact with mammalian keratinous tissue without undue toxicity, incompatibility, instability, allergic response, and the like.

The term "effective amount," as used herein, means an amount of a compound or composition sufficient to increase the diameter of the shafts in the subject region of hair by a statistically significant amount.

The term "result-effective period of time," as used herein, means a period of time sufficient to increase the diameter of the hair shafts in the subject region of hair by a statistically significant amount.

The term "safe and effective amount," as used herein, means an amount of a compound or composition sufficient to increase the diameter of the hair shafts in the subject region of hair by a statistically significant amount, when used for a result-effective period of time, but low enough to avoid serious side effects, i.e., to provide a reasonable benefit to risk ratio, within the scope of sound judgment of the skilled artisan.

Unless otherwise specified, in all embodiments of the present invention, all percentages are by weight of the total composition, unless specifically stated otherwise. All ratios are weight ratios, unless specifically stated otherwise. All ranges are inclusive and combinable. The number of significant digits conveys neither limitations on the indicated amounts nor on the accuracy of the measurements. All numerical amounts are understood to be modified by the word "about" unless otherwise specifically indicated. All measurements are understood to be made at 25°C and at ambient conditions, where "ambient conditions" means conditions under one atmosphere of pressure and at 50% relative humidity.

### A. Hair Care Compositions

In one aspect, the present invention provides hair care compositions that can be used to increase the appearance of thicker and fuller hair. The hair care composition comprises a hair growth inhibiting agent. The hair care composition may comprise two or more hair growth inhibiting agents. Preferably, the hair growth inhibiting agent is present in a safe and effective amount. As used herein, the singular term "hair growth inhibiting agent" is broad enough to include one or a combination of more than one hair growth inhibition agent. Optionally, the hair care compositions can comprise a dermatologically-acceptable carrier and/or any desired suitable optional ingredients.

Any suitable hair growth inhibition agent can be used herein, preferably in a safe and effective amount. The composition comprises a xanthine compound, a vitamin B3 compound, a panthenol compound, or mixtures thereof. Particular materials are described in more detail below.

### 1. Xanthine Compounds

The compositions of the present invention can include a xanthine compound. As used herein, "xanthine compound" means one or more xanthines, derivatives therof, and mixtures thereof. Xanthine Compounds that can be useful herein include, but are not limited to, caffeine, xanthine, 1-methyl xanthine, theophylline, theobromine, derivatives thereof, and mixtures thereof. The composition comprises from 0.1% to 10% of a xanthine compound, preferably from 0.5% to 5% of a xanthine compound, more preferably from 1% to 2% of a xanthine compound.

### 2. Vitamin B₃ Compounds

The compositions of the present invention can include a vitamin B3 compound. Vitamin B3 compounds are particularly useful for regulating skin conditions, as described in U.S. Patent No. 5,939,082. The composition comprises from 0.1% to 25% of a vitamin B3 compound, preferably from 0.5% to 15% of a vitamin B3 compound, more preferably from 3.5% to 7.5% of a vitamin B3 compound. As used herein, "vitamin B3 compound" means a one or more compounds having the formula: 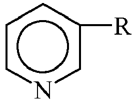 wherein R is - CONH₂ (i.e., niacinamide), - COOH (i.e., nicotinic acid) or - CH2OH (i.e., nicotinyl alcohol); derivatives thereof; mixtures thereof; and salts of any of the foregoing.

Exemplary derivatives of the foregoing vitamin B3 compounds include nicotinic acid esters, including non-vasodilating esters of nicotinic acid (e.g, tocopherol nicotinate, myristyl nicotinate), nicotinyl amino acids, nicotinyl alcohol esters of carboxylic acids, nicotinic acid N-oxide and niacinamide N-oxide.

Suitable esters of nicotinic acid include nicotinic acid esters of C₄-C₂₂, preferably C₁-C₁₆, more preferably C₁-C₆ alcohols. The alcohols are suitably straight-chain or branched chain, cyclic or acyclic, saturated or unsaturated (including aromatic), and substituted or unsubstituted. The esters are preferably non-vasodilating. As used herein, "non-vasodilating" means that the ester does not commonly yield a visible flushing response after application to the skin in the subject compositions (the majority of the general population would not experience a visible flushing response, although such compounds may cause vasodilation not visible to the naked eye, i.e., the ester is non-rubifacient). Non-vasodilating esters of nicotinic acid include tocopherol nicotinate and inositol hexanicotinate; tocopherol nicotinate is preferred.

Other derivatives of the vitamin B₃ compound are derivatives of niacinamide resulting from substitution of one or more of the amide group hydrogens. Nonlimiting examples of derivatives of niacinamide useful herein include nicotinyl amino acids, derived, for example, from the reaction of an activated nicotinic acid compound (e.g., nicotinic acid azide or nicotinyl chloride) with an amino acid, and nicotinyl alcohol esters of organic carboxylic acids (e.g., C1 - C18). Specific examples of such derivatives include nicotinuric acid (C8H8N2O3) and nicotinyl hydroxamic acid (C6H6N2O2), which have the following chemical structures:
nicotinuric acid: 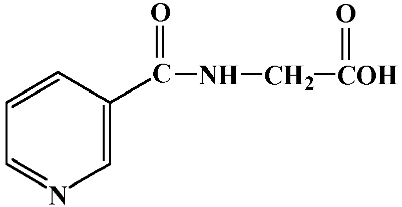
nicotinyl hydroxamic acid:

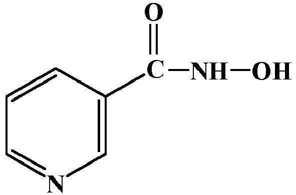

Exemplary nicotinyl alcohol esters include nicotinyl alcohol esters of the carboxylic acids salicylic acid, acetic acid, glycolic acid, palmitic acid and the like. Other non-limiting examples of vitamin B3 compounds useful herein are 2-chloronicotinamide, 6-aminonicotinamide, 6-methylnicotinamide, n-methyl-nicotinamide, n,n-diethylnicotinamide, n-(hydroxymethyl)-nicotinamide, quinolinic acid imide, nicotinanilide, n-benzylnicotinamide, n-ethylnicotinamide, nifenazone, nicotinaldehyde, isonicotinic acid, methyl isonicotinic acid, thionicotinamide, nialamide, 1-(3-pyridylmethyl) urea, 2-mercaptonicotinic acid, nicomol, and niaprazine.

Examples of the above vitamin B3 compounds are well known in the art and are commercially available from a number of sources, e.g., the Sigma Chemical Company (St. Louis, MO); ICN Biomedicals, Inc. (Irvin, CA) and Aldrich Chemical Company (Milwaukee, WI).

One or more vitamin B3 compounds may be used herein. Preferred vitamin B3 compounds are niacinamide and tocopherol nicotinate. Niacinamide is more preferred.

When used, salts, derivatives, and salt derivatives of niacinamide are preferably those having substantially the same efficacy as niacinamide.

Salts of the vitamin B3 compound are also useful herein. Nonlimiting examples of salts of the vitamin B3 compound useful herein include organic or inorganic salts, such as inorganic salts with anionic inorganic species (e.g., chloride, bromide, iodide, carbonate, preferably chloride), and organic carboxylic acid salts (including mono-, di- and tri- C1 - C18 carboxylic acid salts, e.g., acetate, salicylate, glycolate, lactate, malate, citrate, preferably monocarboxylic acid salts such as acetate). These and other salts of the vitamin B3 compound can be readily prepared by the skilled artisan, for example, as described by W. Wenner, "The Reaction of L-Ascorbic and D-Iosascorbic Acid with Nicotinic Acid and Its Amide", J. Organic Chemistry, Vol. 14, 22-26 (1949). Wenner describes the synthesis of the ascorbic acid salt of niacinamide.

The ring nitrogen of the vitamin B3 compound may be substantially chemically free (e.g., unbound and/or unhindered), or after delivery to the skin becomes substantially chemically free ("chemically free" is hereinafter alternatively referred to as "uncomplexed"). More preferably, the vitamin B3 compound is essentially uncomplexed. Therefore, if the composition contains the vitamin B3 compound in a salt or otherwise complexed form, such complex is preferably substantially reversible, more preferably essentially reversible, upon delivery of the composition to the skin. For example, such complex should be substantially reversible at a pH of from 5.0 to 6.0. Such reversibility can be readily determined by one having ordinary skill in the art.

More preferably the vitamin B3 compound is substantially uncomplexed in the composition prior to delivery to the keratinous tissue. Exemplary approaches to minimizing or preventing the formation of undesirable complexes include omission of materials which form substantially irreversible or other complexes with the vitamin B3 compound, pH adjustment, ionic strength adjustment, the use of surfactants, and formulating wherein the vitamin B3 compound and materials which complex therewith are in different phases. Such approaches are well within the level of ordinary skill in the art.

Thus, preferably, the vitamin B3 compound may contain a limited amount of the salt form and is more preferably substantially free of salts of a vitamin B3 compound. Preferably the vitamin B3 compound contains less than 50% of such salt, and is more preferably essentially free of the salt form. The vitamin B3 compound in the compositions hereof having a pH of from 4 to 7 typically contain less than 50% of the salt form.

The vitamin B3 compound may be included as the substantially pure material, or as an extract obtained by suitable physical and/or chemical isolation from natural (e.g., plant) sources. The vitamin B3 compound is preferably substantially pure, more preferably essentially pure.

### 3. Panthenol Compounds

The compositions of the present invention may comprise a panthenol compound. As used herein, the term "panthenol compound" is broad enough to include panthenol, one or more pantothenic acid derivatives, and mixtures thereof. Panthenol and its derivatives can include D-panthenol ([R]-2,4-dihydroxy-N-[3-hydroxypropyl)]-3,3-dimethylbutamide), DL-panthenol, pantothenic acids and their salts, preferably the calcium salt, panthenyl triacetate, royal jelly, panthetine, pantotheine, panthenyl ethyl ether, pangamic acid, pantoyl lactose, Vitamin B complex, or mixtures thereof.

Compositions comprising pantothenic acid derivatives that remain more stable than panthenol and other similar materials in acidic compositions or in compositions containing acid-producing materials such as aluminum-containing actives, can also be suitable for use herein. The selected pantothenic acid derivatives are most typically in liquid form and dispersed throughout or otherwise solubilized within the liquid carrier component of the composition.

The term "pantothenic acid derivative" as used herein refers to those materials that conform to the formula: 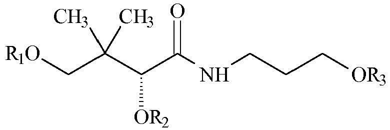 wherein R₁, R₂ and R₃ are hydrogen, C2-C20 hydrocarbons, C2-C20 carboxylic acid esters, or combinations thereof, provided that not more than two of R1, R2 and R3 are hydrogen. R₁, R₂ and R₃ may be independently selected from hydrogen, C2-C8 hydrocarbons, C2-C8 carboxylic acid esters, or combinations thereof. R₁ and R₂ may be hydrogen, and R₃ may be a C2-C8 hydrocarbon, C2-C8 carboxylic acid ester, or combinations thereof. R₁ and R₂ may be hydrogen and R₃ is ethyl. The selected pantothenic acid derivatives may be derived or otherwise obtained from any known source, which may include pantothenic acid or materials other than pantothenic acid, so long as the resulting material has the above defined chemical formula.

Specific non-limiting examples of selected pantothenic acid derivatives for use herein include ethyl panthenol, panthenyl triacetate, and combinations thereof. A pantothenic acid derivative may comprise the d-isomeric form(s) of such derivative form(s), such as d-ethyl panthenol.

### 4. Optional Ingredients

The compositions of the present invention can also additionally comprise any suitable optional ingredients as desired. For example, the composition can optionally include other active or inactive ingredients.

For instance, the present invention may include additional skin care actives selected from the group consisting of sugar amines, retinoids, peptides, dialkanoyl hydroxyproline, hexamidine, salicylic acid, phytosterol, sunscreen actives, water soluble vitamins, oil-soluble vitamins, their derivatives, their precursors, and combinations thereof. Furthermore, the composition may include suitable ingredients that are conventionally used in given product types. The CTFA Cosmetic Ingredient Handbook, Tenth Edition (published by the Cosmetic, Toiletry, and Fragrance Association, Inc., Washington, D.C.) (2004) (hereinafter "CTFA"), describes a wide variety of nonlimiting materials that can be added to the composition herein. Examples of these ingredient classes include, but are not limited to: abrasives, absorbents, aesthetic components such as fragrances, pigments, colorings/colorants, essential oils, skin sensates, astringents, etc. (e.g., clove oil, menthol, camphor, eucalyptus oil, eugenol, menthyl lactate, witch hazel distillate), anti-acne agents, anti-caking agents, antifoaming agents, antimicrobial agents (e.g., iodopropyl butylcarbamate), antioxidants, binders, biological additives, buffering agents, bulking agents, chelating agents, chemical additives, colorants, cosmetic astringents, cosmetic biocides, denaturants, drug astringents, external analgesics, film formers or materials, e.g., polymers, for aiding the film-forming properties and substantivity of the composition (e.g., copolymer of eicosene and vinyl pyrrolidone), opacifying agents, pH adjusters, propellants, reducing agents, sequestrants, skin bleaching and lightening agents, (e.g. hydroquinone, kojic acid, ascorbic acid, magnesiuim ascorbyl phosphate, ascorbyl glucoside, pyridoxine), skin-conditioning agents (e.g. humectants and occlusive agents), skin treating agents (e.g. vitamin D compounds, mono-,di-, and tri-terpenoids, beta-ionol, cedrol), thickeners, hair conditioning agents, and surfactants.

The composition comprises a thickener to increase the substantivity of the composition, such that it does not drip undesirably onto other areas of the body, onto clothing, or onto home furnishings. Preferably. the thickener may be a cellulose-based thickener such as hydroxypropylmethylcellulose. The composition may comprise alcohol and/or water. The composition comprises from 10-90% alcohol, alternatively from 15-75% alcohol, or alternatively from 25-50% alcohol. Any suitable alcohol, such as ethanol, can be used.

The composition may comprise at least one nitrone derivative. Nitrones are capable of irreversibly capturing electrons and/or free radicals, thereby reducing the relative amount of oxidative potential in a microenvironment. Thus, these materials have been referred to as "spin traps" since the ability to detect a free radical via spectroscopic means involves monitoring the spin resonance of free radicals. By irreversibly binding the free radical, the spectroscopic signal becomes reduced due to the free radical becoming trapped by a nitrone such as α-phenyl butyl nitrone (PBN). These can include α-phenyl butyl nitrone (PBN), PBN doxylcyclohexane radicals, 5,5-dimethyl pyrroline N-oxide (DMPO), α-(4-pyridyl 1-oxide)-N-tert-butylnitrone (POBN), 2,2,6,6-tetramethylpiperidine 1-oxide, 4-hydroxytetramethylpiperidine 1-oxide, and the salts of N-(1 -oxido-2,2,6,6-tetramethyl-4-piperidyl)-N,N-dimethyl-N-hydroxyethylammonium, 3,5-dibromo-4-nitrosobenzenesulfonic acid, 2-methyl-2-nitrosopropane, nitrosodisulfonic acid, α-(4-pyridyl-1-oxide)-N-t-butylnitrone, 3,3,5,5-tetramethylpyrroline N-oxide, and 2,4,6-tri-t-butylnitrosobenzene, or spin-trapping derivatives thereof, and mixtures thereof. The spin trap may be PBN.

### C. METHOD FOR INCREASING THE APPEARANCE OF THICKER AND FULLER HAIR

The present invention also provides a method for increasing the diameter of the hair shaft and follicle, leading to an appearance of thicker and/or fuller hair. In one aspect, the method comprises applying a hair care composition comprising a hair growth inhibiting agent to a skin surface from which a region of styled hair grows. For instance, the hair care composition can be applied to the scalp and/or face (e.g., beard or moustache area). The method comprises topically applying a hair care composition comprising an effective amount of a hair growth inhibiting agent to a region of skin of a mammal seeking to increase the appearance of thicker and/or fuller hair. The invention can provide use of a hair care composition comprising a hair growth reduction agent for increasing the diameter of the hair shaft and/or follicle.

The region of hair can be located on any part of the body. For instance, it can grow from a skin surface located on at least a portion of the scalp or the face.

The method may comprise applying the composition according to a regimen, wherein said regimen comprises:
(a) cleansing the scalp and/or face to form a cleansed scalp and/or face;
(b) topically applying the composition to said cleansed scalp and/or cleansed face.

### EXAMPLES

The following are non-limiting examples of the present invention. The examples are given solely for the purpose of illustration and are not to be construed as limitations of the present invention, as many variations thereof are possible without departing from the spirit and scope of the invention, which would be recognized by one of ordinary skill in the art.

In the examples, all concentrations are listed as weight percent, unless otherwise specified and may exclude minor materials such as diluents, filler, and so forth. The listed formulations, therefore, comprise the listed components and any minor materials associated with such components. As is apparent to one of ordinary skill in the art, the selection of these minors will vary depending on the physical and chemical characteristics of the particular ingredients selected to make the present invention as described herein.

**Examples 1-4: Shampoo examples**

| **Component** | **1 (wt%)** | **2 (wt%)** | **3 (wt%)** | **4 (wt%)** |
|---|---|---|---|---|
| Water | Q.S. | Q.S. | Q.S. | Q.S. |
| Rheology modifying system, anionic polymer MVE/MA crosslinked copolymer (Stabileze 06) | 0.05 | 0.05 | - | - |
| Rheology modifying system, clay Hydrous Na, Mg silicate (Laponite XLS) | 0.05 | 0.05 | 0.05 | - |
| Hydroxypropyl methylcellulose (PrimaFlo) | - | - | 0.10 | - |
| Polyquaternium 10 (Ucare Polymer LR-400) | 0.50 | 0.50 | 0.50 | 0.50 |
| Coconut monoethanolamide (Monamid CMA) | 1.09 | 1.03 | 1.03 | 1.50 |
| Disodium EDTA (Disslovine Na₂S) | 0.14 | 0.14 | 0.14 | 0.10 |
| Sodium Benzoate (Purox S Grains) | 0.25 | 0.25 | 0.25 | 0.25 |
| Sodium Citrate Dihydrate | 0.45 | 0.45 | 0.45 | 0.45 |
| Sodium Laureth-3-Sulfate (SLE3S) | 2.18 | - | - | - |
| Cocamidopropyl Betaine (Tegobetaine F-B) | 2.18 | - | - | - |
| Sodium lauryl sulfate (SLS) | 6.55 | - | - | - |
| Citric Acid | 0.08 | - | - | 0.04 |
| BHT | 0.50 | 0.50 | 0.50 | 0.50 |
| Sodium Chloride | 0.25 | 0.75 | 0.50 | 0.01 |
| Sodium Hydroxide | 0.01 | - | - | - |
| Dimethicone (Viscasil 3000,000) | 1.35 | 1.35 | 1.35 | 1.35 |
| Ammonium Laureth-3-Sulfate (AE3S) | 0.07 | 4.11 | 6.00 | 6.00 |
| Ethylene glycol distearate (EGDS) | 1.50 | 1.50 | 1.50 | 1.50 |
| Ammonium Lauryl Sulfate (ALS) | 1.50 | 6.88 | 6.88 | 10.00 |
| Hexamidine diisethionate | 0.10 | 0.10 | 0.10 | 0.10 |
| Glyceryl dilaurate | 2.00 | 2.00 | 2.00 | 2.00 |
| Methylchloroisothiazolinone & Methylisothiazolinone (Kathon CG) | 0.0005 | 0.0005 | 0.0005 | 0.0005 |
| Fragrance | 0.70 | 0.70 | 0.70 | 0.70 |
| PEG 7M (Polyox WSR-N-750) | 0.10 | - | - | 0.10 |
| DL Panthenol 50% soln. (DL-Panthenol 50L) | 0.03 | 0.03 | 0.03 | 0.03 |
| DL Panthenyl Ethyl Ether (Pantyl Ethyl Ether) | 0.03 | 0.03 | 0.03 | 0.03 |
| Lysine Monochloride | 0.03 | 0.03 | 0.03 | 0.03 |
| L-Tyrosine Methylester Hydrochloride (Methyl Tyrosine) | 0.01 | 0.01 | 0.01 | 0.01 |
| Histidine | 0.01 | 0.01 | 0.01 | 0.01 |
| Cetyl Alcohol | ----- | ----- | | 0.90 |

**Example 5-7: Conditioner examples**

| **Component** | **5 (wt%)** | **6 (wt%)** | **7 (wt%)** |
|---|---|---|---|
| Dimethicone compound-1 *1 | - | 4.20 | |
| Dimethicone compound-2 ^{*}2 | - | - | 2.00 |
| Silicone compound-2 *3 | 3.50 | - | |
| Behenyl trimethyl ammonium chloride *6 | 2.25 | - | 3.38 |
| Isopropyl alcohol | 0.60 | - | 0.90 |
| Stearamidopropyl dimethylamine *7 | - | 2.00 | - |
| Glutamic acid *8 | - | 0.64 | - |
| Cetyl alcohol *9 | 1.90 | 2.50 | 2.30 |
| Stearyl alcohol *10 | 4.60 | 4.50 | 4.20 |
| Polysorbate-20 *11 | - | - | - |
| PPG-34 *12 | - | - | - |
| Polyalphaolefin *13 | - | - | - |
| BHT | 0.50 | 0.50 | 0.50 |
| Benzyl alcohol | 0.40 | 0.40 | 0.40 |
| Glyceryl dilaurate | 2.00 | 2.00 | 2.00 |
| Methylchloroisothiazolinon e/ Methylisothiazolinone *14 | 0.0005 | 0.0005 | 0.0005 |
| Perfume | 0.35 | 0.50 | 0.35 |
| NaOH | 0.014 | - | 0.014 |
| Panthenol *15 | 0.05 | - | 0.05 |
| Panthenyl ethyl ether *16 | 0.05 | - | 0.05 |
| Hexamidine diisethionate | 0.10 | 0.10 | 0.10 |
| Hydrolyzed collagen *17 | - | 0.01 | - |
| Vitamin E *18 | - | 0.01 | - |
| Decyl Glucoside *19 | - | - | - |
| Octyl methoxycinnamate | - | 0.09 | - |
| Benzophenone-3 | - | 0.09 | - |
| Disodium EDTA | 0.13 | 0.13 | 0.13 |
| Deionized water | Qs | Qs | Qs |

### Definitions of Components

*1 Dimethicone/Cyclomethicone: a blend dimethicone having a viscosity of 18,000,000mPas and cyclopentasiloxane available from GE Toshiba
*2 Dimethicone blend: a blend of dimethicone having a viscosity of 18,000,000mPas and dimethicone having a viscosity of 200mPas available from GE Toshiba
*3 Available from GE having a viscosity 10,000mPas, and having following formula (I): (R₁)ₐG₃₋ₐ-Si-(-OSiG₂)ₙ-(-OSiG_{b}(R₁)_{2-b})ₘ-O-SiG₃₋ₐ(R₁)ₐ (I)
   wherein G is methyl; a is an integer of 1; b is 0, 1 or 2, preferably 1; n is a number from 400 to 600; m is an integer of 0; R₁ is a monovalent radical conforming to the general formula C_{q}H_{2q}L, wherein q is an integer of 3 and L is -N(CH₃)₂
*6 Behenyl trimethyl ammonium chloride/Isopropyl alcohol: Genamin KDMP available from Clariant
*7 Stearamidopropyl dimethylamine: Lexamine S-13 available from Inolex
*8 Glutamic acid: available from Ajinomoto
*9 Cetyl alcohol: Konol series available from Shin Nihon Rika.
*10 Stearyl alcohol: Konol series available from Shin Nihon Rika.
*11 Polysorbate-20: Glycosperse L-20K available from Lonza Inc.
*12 PPG-34: New Pol PP-2000 available from Sanyo Kasei.
*13 Polyalphaolefin: PureSyn 100 available from ExxonMobil Chemical Company
*14 Methylchloroisothiazolinone/Methylisothiazolinone: Kathon CG available from Rohm & Haas
*15 Panthenol: Available from Roche.
*16 Panthenyl ethyl ether: Available from Roche.
*17 Hydrolyzed collagen: Peptein 2000 available from Hormel.
*18 Vitamin E: Emix-d available from Eisai.
*19 Decyl glucoside: Plantacare 2000UP available from Cognis Japan Ltd.

**Example 8: Tonic example**

| **Component** | **8 (wt%)** |
|---|---|
| Alcohol 100% DEB 100 (Ethanol) | 25.00 |
| Carbomer (Carbopol Ultrez 10) | 0.10 |
| Hexamidine diisethionate | 0.10 |
| Glyceryl dilaurate | 2.00 |
| BHT | 0.50 |
| Triethanolamine | 0.20 |
| Caffeine | 1.50 |
| Niacinamide | 5.00 |
| Panthenol | 0.30 |
| Deionized water | Qs |

**Example 9: Common base for dyeing example**

| **Component** | **9 (wt%)** |
|---|---|
| Propylene glycol | 9.50 |
| Ammonium hydroxide | 5.00 |
| Ethoxydiglycol | 4.00 |
| Ethanolamine | 4.50 |
| Oleic acid | 1.00 |
| Hexylene glycol | 6.00 |
| Hexamidine diisethionate | 0.10 |
| Glyceryl dilaurate | 2.00 |
| BHT | 0.50 |
| Cocamidopropyl betaine | 3.50 |
| Oleth-10 | 0.30 |
| Oleth-2 | 0.30 |
| Dilinoleic acid | 1.50 |
| C12-C15 Pareth-3 | 0.50 |
| Soytrimonium chloride | 7.00 |
| Sodium metasilicate | 0.05 |
| Erythorbic acid | 0.50 |
| EDTA | 0.03 |
| Sodium sulfite | 0.30 |
| 1 -Phenyl-3-methyl-5-pyrazolone | 0.20 |
| Deionized water | Qs |

**Example 10: Common base for mousse**

| **Component** | **10 (wt%)** |
|---|---|
| Ethanol | 51.80 |
| Propylene glycol | 5.00 |
| Propellant P75 | 4.30 |
| Cetyl alcohol | 2.20 |
| Glyceryl dilaurate | 2.00 |
| Stearyl alcohol | 1.00 |
| Polyoxyethylene lauryl alcohol | 1.00 |
| BHT | 0.50 |
| Polysorbate 60 | 0.40 |
| Hexamidine diisethionate | 0.10 |
| Acetic acid | Qs pH 6.0 |
| Deionized water | Qs |

### Example 11: Method of Marketing

The shampoo of Example 1 is packaged into a blue and white container and offered for sale to consumers at a retail store. A label on the container communicates that when this shampoo is used to wash hair, it will help to increase the appearance of thicker and fuller hair.

### Example 12: Method of Marketing

A shampoo contained in a blue and white bottle (herein "Subject Shampoo") is located on a shelf next to the shampoo of Example 11. A label is attached to the Subject Shampoo's bottle which directs the consumer to compare the Subject Shampoo to the shampoo of Example 11.

### Example 13: Clinical Study

A composition comprising a mixture of Caffeine, Niacinamide and Panthenol demonstrated a clinically significant increase in hair width versus a placebo. In this study, a scalp tonic containing 5% Niacinamide, 1.5% Caffeine, and 0.3% D-Panthenol in a water/alcohol matrix (25% ethanol, q.s. water) was evaluated. This formula also included 0.3% hydroxypropylmethylcellulose as a thickening agent. In this split-head study, 1.5 ml of the tonic was applied to one side of the head two times per day, and 1.5ml of the placebo was applied to the other side of the head two times daily.

This was a fourteen week, double blinded, randomized, split-head and controlled clinical study in women ages 18-65 (inclusive) who perceived themselves as having thinning hair. This study consisted of a 2 week pre-treatment phase (use of placebo on both sides of the head to learn proper product application methods prior to the actual treatment phase) followed by a 12 week treatment phase.

Upon arriving at the test site, subjects were acclimated in an environmentally controlled room (70°F ± 2 and 30-45% relative humidity) for 30 minutes prior to having their scalp evaluated by a qualified skin grader to assess skin health. After subjects had their scalp assessed by a qualified skin grader, a licensed cosmetologist identified two scalp sites for clipping (areas of approximately 1.8 cm x 1.8 cm), one on the top left lateral scalp, approximately 4-5" back into the hairline and the other was symmetrically placed on the top right lateral scalp. A template was used to mark the 4 corners and a notch slightly below center of each treatment site. The clipped sites were approximately 2" lateral to either of their respective sides from the midline of the head/scalp. The licensed cosmetologist then cut the hair within each of the scalp clipping sites with a small battery operated hair clipper to 1mm in length. After the sites were prepared, a trained clinical assistant placed a small, permanent dot tattoo approximately 1 mm above the marked notch (located slightly below center of the treatment site) on each treatment site to provide a reference for identification of these sites for digital imaging that was performed at designated study time points.

For Baseline, Week 4, 8, and 12, the clipped scalp sites were identified using the template and marking the 4 corners of the template with a Sharpie® marker or equivalent. The licensed cosmetologist then evenly clipped the short hairs within the 2 clipped sites to 1 mm as previously conducted. Following hair clipping, a technician captured a baseline image of each clipped scalp site using a Hi-Scope® digitized imaging system. The tattoo that was placed within each lower treatment site was used as a reference point to position the image for capture and storage. This baseline image was used when blending post-treatment images during the study. The next day, approximately 24 hours following the hair clipping visit, a technician captured an image of each lower scalp clipping site using a Hi-Scope® digitized imaging system. The tattoo that was placed within each lower treatment site was used as a reference point to position the image for capture and storage after aligning the hair follicle openings with the image captured at Baseline, Day 1. This procedure was used for Week 4, 8 and 12.

A statistically significant increase in hair diameter was observed for the hair tonic treated hair vs. the placebo-treated hair in the human clinical test. The Change from Baseline Adjusted Mean for the tonic-treated hair is larger than that of the placebo-treated, with the results being statistically significant (p=0.0466). As this is a split-head design, subject variation was greatly minimized. Additionally, since the exact same hairs were imaged throughout the study, variations in width due to measuring different hairs through the study were greatly minimized.

**Example 13: Clinical Study- Comparison of treatment effect**

| N | Treatment | Grouping | Change from Baseline Adjusted Mean (µm) | P value |
|---|---|---|---|---|
| 40 | Placebo (Tonic Vehicle) | A | 0.165 | ---------- |
| 40 | Tonic Product | B | 0.663 | 0.0466* |

| | | | | |
|---|---|---|---|---|
| * one-tail test | | | | |

An image analysis algorithm was used to measure the hair diameter from the digital micrographs. The algorithm automatically carried out several steps to isolate and characterize hairs in each digital image. First, the algorithm performed a local background leveling function and enhancement of the red component in the color image. Automatic thresholding of the color image was performed to obtain a binary image (black and white) of the hairs on the background. The algorithm then identified hairs as continuous black lines and broke the lines into individual hairs at branch points and points of high curvature. Next, the algorithm eliminated very short objects and retained the remaining objects as 'hairs'. In the final step, the algorithm measured and recorded position and diameter (width) of each detected hair.

The hair system image acquisition and analysis methods used herein were the methods published by Berger, R.S. et al., in 2003 in "The effects of minoxidil, 1% pyrithione zinc and a combination of both on hair density: a randomized controlled trial. British Journal of Dermatology, 149, 354-362," with the exception that for the study of this example, different treatments, scalp imaging sites, and timepoints were used.

### Example 14: Synergy Example.

An in vitro model, consisting of primary human dermal papilla cells in culture, demonstrates a surprising synergistic increase in the survival of metabolically stressed dermal papilla cells. Primary human dermal papilla cells were metabolically stressed for 48 hours in reduced standard tissue culture media. During this period of stress the cells were treated with the mixtures below. After 48 hr the metabolic activity of the cells was measured by the amount of ATP using the Cell Titer Glo™ kit (Promega).

While the individual components provided little or no benefit in increasing the survival of stressed dermal papilla cells, the mixture of the three components resulted in a synergistic effect that produced a statistically significant increase in cell survival, not only above the calculated average but also above the expected additive effect of the individual components. Thus, the investigators demonstrated that the combination provided a significantly greater increase in cell survival than one would expect, given the effects of the individual components alone or in addition to one another. The dermal papilla, in normal human hair, is the control center for hair diameter. Increasing survival of dermal papilla cells in situ leads to increased hair diameter. Accordingly, an increase in survival of dermal papilla cells correlates with an increase in hair diameter.

| Treatment | Survival | *p* value versus calculated mixture |
|---|---|---|
| Vehicle | 1.00 | |
| Niacinamide (1.5 mM) | 0.71 | |
| Panthenol (0.03%) | 1.02 | |
| Caffeine (0.02%) | 0.94 | |
| Calculated Average for the Mixture | 0.89 | |
| Observed Survival Rate for the Mixture | 1.14 | *p* < 0.05 |
| | | |
| | | |
| Vehicle | 1.00 | |
| Niacinamide (0.5 mM) | 1.02 | |
| Panthenol (0.3%) | 0.75 | |
| Caffeine (0.05%) | 0.98 | |
| Calculated Average for the Mixture | 0.92 | |
| Observed Survival Rate for the Mixture | 1.38 | *p* < 0.05 |

## Claims

1. A hair care composition for increasing the hair shaft diameter to give the appearance of thicker and fuller hair, comprising a safe and effective amount of;
a. a xanthine compound, preferably caffeine;
b. a vitamin B3 compound, preferably niacinamide; and
c. a panthenol compound, preferably panthenol.
wherein said composition comprises:
a. from 1% to 2% caffeine;
b. from 3.5% to 7.5% niacinamide;
c. from 0.2% to 0.5% panthenol; and
d. a thickener, preferably wherein said thickener is cellulose-based, more preferably hydroxypropylmethylcellulose;
e. from 10-90% alcohol, preferably 15-75% alcohol, more preferably from 25-50% alcohol, wherein said alcohol is ethanol;
f. water.

2. The composition according to claim 1, further comprising at least one nitrone derivative selected from the group consisting of α-phenyl butyl nitrone (PBN), PBN doxylcyclohexane radicals, 5,5-dimethyl pyrroline N-oxide (DMPO), α-(4-pyridyl 4-oxide)-N-tert-butylnitrone (POBN), 2,2,6,6-tetramethylpiperidine 1-oxide, 4-hydroxytetramethylpiperidine 1-oxide, and the salts of N-(1-oxido-2,2,6,6-tetramethyl-4-piperidyl)-N,N-dimethyl-N-hydroxyethylammonium, 3,5-dibromo-4-nitrosobenzene sulfonic acid, 2-methyl-2-nitrosopropane, nitrosodisulfonic acid, α-(4-pyridyl-1-oxide)-N-t-butylnitrone, 3,3,5,5-tetramethylpyrroline N-oxide, and 2,4,6-tri-tbutylnitrosobenzene, or spin-trapping derivatives thereof, and mixtures thereof.

3. The composition according to any of the preceding claims, wherein the composition is a tonic having the following composition:
| **Component** | **(wt%)** |
|---|---|
| Alcohol 100% DEB 100 (Ethanol) | 25.00 |
| Carbomer (Carbopol Ultrez 10) | 0.10 |
| Hexamidine diisetbionate | 0.10 |
| Glyceryl dilaurate | 2.00 |
| BHT | 0.50 |
| Triethano lamine | 0.20 |
| Caffeine | 1.50 |
| Niacinamide | 5.00 |
| Panthenol | 0.30 |
| Deionized water | Qs |

4. A method for increasing hair shaft diameter to give the appearance of thicker and fuller hair, comprising topically applying the composition of any of claims 1-3 to a region where thicker or fuller hair is desired.

5. A method according to claim 4, wherein the composition is applied to the scalp and/or face.

6. A method, according to claims 4 or 5, comprising applying a composition according to a regimen, wherein said regimen comprises:
(a) cleansing the scalp and/or face to form a cleansed scalp and/or face;
(b) topically applying the composition according to any of claims 1 to 3 to said cleansed scalp and/or cleansed face.

7. Use of the composition according to any of claims 1-3 for increasing the hair shaft diameter to give the appearance of thicker and fuller hair.

## Patentansprüche

1. Haarpflegezusammensetzung zur Vergrößerung eines Haarschaftdurchmessers, um Haar dicker und voller erscheinen zu lassen, die eine sichere und wirksame Menge an Folgendem aufweist:
a. einer Xanthinverbindung, vorzugsweise Koffein;
b. einer Vitamin-B3-Verbindung, vorzugsweise Niacinamid; und
c. einer Panthenolverbindung, vorzugsweise Panthenol,
wobei die Zusammensetzung Folgendes umfasst:
a. 1 % bis 2 % Koffein;
b. 3,5 % bis 7,5 % Niacinamid;
c. 0,2 % bis 0,5 % Panthenol; und
d. ein Verdickungsmittel, wobei das Verdickungsmittel vorzugsweise auf Cellulose, stärker bevorzugt auf Hydroxypropylmethylcellulose basiert;
e. 10-90 % Alkohol, vorzugsweise 15-75 % Alkohol, mehr bevorzugt 25-50 % Alkohol, wobei der Alkohol Ethanol ist;
f. Wasser.

2. Zusammensetzung nach Anspruch 1, die ferner mindestens ein Nitronderivat umfasst, das ausgewählt ist aus der Gruppe bestehend aus α-Phenylbutylnitron (PBN), PBN-Doxylcyclohexanresten, 5,5-Dimethylpyrrolin-N-oxid (DMPO), α-(4-Pyridyl-4-oxid)-N-tert-butylnitron (POBN), 2,2,6,6-Tetramethylpiperdin-1-oxid, 4-Hydroxytetramethylpiperidin-1-oxid und den Salzen von N-(1-Oxido-2,2,6,6-tetramethyl-4-piperidyl)-N,N-dimethyl-N-hydroxyethylammonium, 3,5-Dibrom-4-nitrosobenzolsulfonsäure, 2-Methyl-2-nitrosopropan, Nitrosodisulfonsäure, α-(4-Pyridyl-1-oxid)-N-t-butylnitron, 3,3,5,5-Tetramethylpyrrolin-N-oxid und 2,4,6-Tri-t-butylnitrosobenzol oder Spin-Trap-Derivaten davon und Mischungen davon.

3. Zusammensetzung nach einem der vorstehenden Ansprüche, wobei die Zusammensetzung ein Kräftigungsmittel mit der folgenden Zusammensetzung ist:
| **Komponente** | **(Gew.-%)** |
|---|---|
| Alkohol 100 % DEB 100 (Ethanol) | 25,00 |
| Carbomer (Carbopol Ultrez 10) | 0,10 |
| Hexamidindiisethionat | 0,10 |
| Glyceryldilaurat | 2,00 |
| BHT | 0,50 |
| Triethanolamin | 0,20 |
| Koffein | 1,50 |
| Niacinamid | 5,00 |
| Panthenol | 0,30 |
| Entionisiertes Wasser | Qs |

4. Verfahren zur Vergrößerung eines Haarschaftdurchmessers, um Haar dicker und voller erscheinen zu lassen, das das topische Auftragen der Zusammensetzung nach einem der Ansprüche 1-3 auf eine Region, wo dickeres oder volleres Haar gewünscht wird, umfasst.

5. Verfahren nach Anspruch 4, wobei die Zusammensetzung auf die Kopfhaut und/oder das Gesicht aufgetragen wird.

6. Verfahren nach Anspruch 4 oder 5, das das Auftragen der Zusammensetzung gemäß Vorgaben umfasst, wobei die Vorgaben Folgendes umfassen:
(a) Reinigen der Kopfhaut und/oder des Gesichts, um eine gereinigte Kopfhaut und/oder ein gereinigtes Gesicht zu erhalten;
(b) topisches Auftragen der Zusammensetzung nach einem der Ansprüche 1 bis 3 auf die gereinigte Kopfhaut und/oder das gereinigte Gesicht.

7. Verwendung der Zusammensetzung nach einem der Ansprüche 1-3 zur Vergrößerung des Haarschaftdurchmessers, um das Haar dicker und voller erscheinen zu lassen.

## Revendications

1. Composition pour soins capillaires pour augmenter le diamètre de tige capillaire pour donner l'apparence de cheveux plus épais et plus fournis, comprenant une quantité sûre et efficace de :
a. un composé de xanthine, de préférence de la caféine ;
b. un composé de vitamine B3, de préférence du niacinamide ; et
c. un composé de panthénol, de préférence du panthénol.
où ladite composition comprend :
a. de 1 % à 2 % de caféine ;
b. de 3,5 % à 7,5 % de niacinamide ;
c. de 0,2 % à 0,5 % de panthénol ; et
d. un épaississant, de préférence dans laquelle ledit épaississant est à base de cellulose, plus préférablement de l'hydroxypropylméthylcellulose ;
e. de 10 à 90 % d'alcool, de préférence 15 à 75 % d'alcool, plus préférablement de 25 à 50 % d'alcool, dans laquelle ledit alcool est l'éthanol ;
f. de l'eau

2. Composition selon la revendication 1, comprenant en outre au moins un dérivé nitrone choisi dans le groupe constitué de α-phényl-butyl-nitrone (PBN), radicaux PBN doxylcyclohexane, N-oxyde de 5,5-diméthyl-pyrroline (DMPO), α-(4-pyridyl 4-oxyde)-N-tert-butylnitrone (POBN), 1-oxyde de 2,2,6,6-tétraméthylpipérdine, 1-oxyde de 4-hydroxytétraméthylpipéridine, et les sels de N-(1-oxido-2,2,6,6-tétaméthyl-4-pipéridyl)-N,N-diméthyl-N-hydroxyéthylammonium, d'acide 3,5-dibromo-4-nitrosobenzène sulfonique, de 2-méthyl-2-nitrosopropane, d'acide nitrosodisulfonique, de α-(4-pyridyl-1-oxyde)-N-t-butylnitrone, de N-oxyde de 3,3,5,5-tétraméthylpyrroline, et de 2,4,6-tritbutylnitrosobenzène, ou de dérivés de piégeage de spin de ceux-ci, et des mélanges de ceux-ci.

3. Composition selon l'une quelconque des revendications précédentes, où la composition est un tonic ayant la composition suivante :
| **Composant** | **(% en poids)** |
|---|---|
| Alcool 100 % DEB 100 (éthanol) | 25,00 |
| Carbomère (Carbopol Ultrez 10) | 0,10 |
| Diiséthionate d'hexamidine | 0,10 |
| Dilaurate de glycéryle | 2,00 |
| BHT | 0,50 |
| Triéthanolamine | 0,20 |
| Caféine | 1,50 |
| Niacinamide | 5,00 |
| Panthénol | 0,30 |
| Eau désionisée | Q.S.P. |

4. Procédé pour augmenter le diamètre de tige capillaire pour donner l'apparence de cheveux plus épais et plus fournis, comprenant l'application locale de la composition selon l'une quelconque des revendications 1 à 3 sur une région où des cheveux plus épais ou plus fournis sont souhaités.

5. Procédé selon la revendication 4, dans lequel la composition est appliquée sur le cuir chevelu et/ou le visage.

6. Procédé, selon les revendications 4 à 5, comprenant l'application d'une composition selon un régime, dans lequel ledit régime comprend :
(a) le nettoyage du cuir chevelu et/ou du visage pour former un cuir chevelu et/ou un visage nettoyés ;
(b) l'application locale de la composition selon l'une quelconque des revendications 1 à 3 sur le(s)dit(s) cuir chevelu et/ou visage nettoyés.

7. Utilisation de la composition selon l'une quelconque des revendications 1 à 3 pour augmenter le diamètre de tige capillaire pour donner l'apparence de cheveux plus épais et plus fournis.
